# FASCICULE DE BREVET EUROPEEN

(11) **EP 2 291 109 B1**
(45) Date de publication et mention de la délivrance du brevet: **11.07.2012**
(21) Numéro de dépôt: 09766043.5
(22) Date de dépôt: 20.05.2009
(51) Int. Cl.: A61B 1/12

(54) **SYSTEME ET PROCEDE POUR STOCKER UN ENDOSCOPE MEDICAL.**
SYSTEM UND VERFAHREN ZUR AUFBEWAHRUNG EINES MEDIZINISCHEN ENDOSKOPS
SYSTEM AND METHOD FOR STORING A MEDICAL ENDOSCOPE

(30) Priorité: 23.05.2008 FR 0853384
(43) Date de publication de la demande: 09.03.2011
(73) Titulaire: Soluscope, 13010 Marseille (FR)
(72) Inventeur: MARIOTTI, Bernard, F-13007 Marseille (FR)
(74) Mandataire: Roman, Alexis
(86) Numéro de dépôt international: PCT/FR2009/050942
(87) Numéro de publication internationale: WO 2009/153481

(56) Documents cités:
- US-A- 5 279 799
- US-A1- 2004 091 391
- US-A1- 2005 000 553

## Description

### Domaine technique de l'invention.

La présente invention concerne un système et un procédé pour stocker un endoscope médical, en particulier après que ce dernier ait été désinfecté à l'aide d'une solution désinfectante.

L'invention concerne le domaine technique général des équipements permettant de traiter un endoscope médical usagé en vue de le rendre réutilisable.

### État de la technique.

Les endoscopes souples utilisés en milieu hospitalier doivent être désinfectés entre deux patients. La désinfection consiste généralement à immerger l'endoscope dans une solution désinfectante ainsi qu'à irriguer avec la même solution les canaux dont il est équipé. Pour cela, des tubulures spécifiques sont utilisées. Elles comprennent à l'une des extrémités un raccord spécifique au type de canal sur lequel elles doivent se raccorder, sur l'autre extrémité un raccord standardisé identique pour chacune des tubulures.

Après leur désinfection, qu'elle soit manuelle dans une paillasse, ou automatique au moyen d'un laveur/désinfecteur (ci-après LDE), les endoscopes médicaux sont stockés en attendant d'être réutilisés.

Les endoscopes médicaux sont équipés de très petits canaux internes qui ne peuvent être convenablement séchés après chaque désinfection en raison du temps que cela peut prendre (plus d'une heure pour un séchage parfait). Durant la phase de stockage (qui peut aller de quelques heures à plusieurs jours), l'endoscope peut alors se recontaminer par colonisation de germes hydriques, soit non éradiqués durant la désinfection, soit issus des manipulations ou des lieux de stockage. Pour répondre à ce problème, des armoires de stockage ont été développées. Elles permettent de faire pendre les endoscopes pour les égoutter. Quelques modèles présentent en supplément une ventilation externe et/ou une irrigation des canaux avec de l'air comprimé.

Les problèmes essentiels posés par ces armoires sont les suivants :
- les manipulations pour déconnecter l'endoscope d'un LDE ou d'une paillasse puis pour le reconnecter de nouveau dans une armoire de stockage sont longues, fastidieuses, sujettes à erreurs et à contamination ;
- les endoscopes ne sont pas protégés les uns des autres et peuvent rentrer en contact soit entre eux, soit avec les bords de l'armoire et ainsi être à l'origine d'infections croisées ;
- les tubulures de raccordement destinées à irriguer les canaux avec de l'air ne sont pas désinfectées entre chaque utilisation, ce qui peut être à l'origine d'une contamination durant le stockage.

Pour tenter de résoudre ces problèmes, on connait par le document brevet EP 0.986.988 (JOHANNES), un système pour sécher un endoscope médical. Ce système comporte une armoire de stockage pourvue d'au moins une étagère sur laquelle est inséré le panier dans lequel est placé l'endoscope. Une fois que le panier est mis en place sur une étagère, les canaux de l'endoscope sont raccordés à un dispositif d'injection d'air sous pression. Plus particulièrement, les canaux de l'endoscope sont reliés à un bloc de connexion agencé en position fixe dans le panier et susceptible de se connecter à différents dispositifs de traitement dont le dispositif d'injection d'air sous pression. L'utilisateur peut ainsi positionner le panier dans un système de nettoyage puis dans un système de désinfection puis dans un système de stockage/séchage, sans avoir à débrancher les canaux de l'endoscope du bloc de connexion.

Le système décrit dans le document EP 0.986.988 ne permet toutefois pas de résoudre l'ensemble des problèmes susmentionnés. En effet, lorsque les endoscopes sont disposés dans leur panier, ils ne sont toujours pas protégés les uns des autres. Des égouttures souillées provenant d'un endoscope peuvent s'égoutter sur un autre endoscope placé à un étage inférieur et être ainsi à l'origine d'infections croisées. En outre, le séchage externe de l'endoscope n'est pas optimal. Un autre inconvénient réside dans le fait que l'endoscope n'est pas protégé lors de son transport depuis le dispositif de désinfection jusque dans l'armoire de stockage.

Face à cet état des choses, le problème technique principal que vise à résoudre l'invention est de perfectionner le système de stockage décrit dans le document EP 0.986.988, en vue de travailler dans des conditions plus hygiéniques et de réduire au minimum les risques de contamination des endoscopes.

### Divulgation de l'invention.

La solution proposée par l'invention est un système pour stocker un endoscope médical, ledit système comportant :
- un bac transportable dans lequel est placé l'endoscope,
- une armoire de stockage pourvue d'au moins une étagère sur laquelle est inséré le bac dans lequel est placé l'endoscope,
- un premier dispositif d'injection d'air raccordé aux canaux de l'endoscope de manière à ce que de l'air sous pression soit injecté dans lesdits canaux lorsque le bac est mis en place sur l'étagère.

Conformément à l'invention, ce système est remarquable en ce que le bac est fermé et en ce que ledit bac comporte :
- un orifice d'entrée se positionnant en vis-à-vis d'un second dispositif d'injection d'air sous pression lorsque ledit bac est mis en place sur l'étagère,
- un orifice de sortie par lequel s'évacue l'air injecté, le flux d'air circulant dans le bac, à l'extérieur de l'endoscope, depuis l'orifice d'entrée jusqu'au dit orifice de sortie, de manière à accélérer le séchage externe dudit endoscope.

Le bac étant fermé, l'endoscope est totalement protégé des autres endoscopes, aucune souillure ne pouvant passer d'un bac à un autre. En outre, le séchage externe et individualisé de l'endoscope permet de mieux le sécher et donc de réduire le développement des infections lors de son stockage. Enfin, la mise sous pression du bac évite que des fluides contaminés puissent pénétrer à l'intérieur dudit bac. Ces caractéristiques agissent en synergies de manière à pouvoir sécher et stocker les endoscopes dans des conditions extrêmement hygiéniques, avec des risques de contamination quasi nuls.

Afin de réduire le volume d'air nécessaire au séchage externe de l'endoscope, l'orifice de sortie du bac débouche avantageusement dans une cavité dans laquelle le flux d'air sous pression est récupéré par le second dispositif d'injection et réinjecté au niveau de l'orifice d'entrée dudit bac.

Selon un mode préféré de réalisation, la cavité est intégrée dans l'étagère sur laquelle est positionné le bac, l'orifice de sortie dudit bac se positionnant en vis à vis d'un orifice d'entrée de ladite cavité lorsque ledit bac est mis en place sur ladite étagère.

Dans une variante de réalisation, la cavité est directement intégrée dans le bac.

Pour éviter de réinjecter de l'air souillé, la cavité peut être pourvue d'un moyen pour stériliser le flux d'air avant sa reprise par le second dispositif d'injection.

Selon une autre caractéristique avantageuse de l'invention permettant de connecter automatiquement les canaux de l'endoscope au premier dispositif d'injection d'air, lesdits canaux sont reliés à un bloc de connexion agencé en position fixe dans le bac, ledit premier dispositif d'injection étant pourvu d'une tête d'injection se connectant automatiquement audit bloc de connexion lorsque ledit bac est mis en place sur l'étagère.

Pour simplifier la conception, le bloc de connexion est disposé sur le fond du bac et la tête d'injection est disposée en saillie sur la face supérieure de l'étagère sur laquelle repose ledit bac.

Pour limiter les risques d'infections croisées, l'armoire de stockage comporte préférentiellement plusieurs étagères destinées à recevoir chacune un bac, chaque bac étant dédié à un endoscope et chaque étagère étant dédiée à un bac.

Les étagères peuvent être pourvues d'un moyen de détrompage mécanique et/ou d'un moyen d'identification permettant d'empêcher ou d'avertir qu'un bac n'est pas positionné sur l'étagère qui lui est dédiée.

Chaque étage de l'armoire de stockage est préférentiellement alimenté de manière cyclique par le premier dispositif d'injection d'air.

Chaque étage de l'armoire est préférentiellement pourvu d'un second dispositif d'injection d'air destiné à faire circuler un flux d'air sous pression dans les bacs fermés, lesdits seconds dispositifs d'injection d'air communiquant avec une même colonne d'air filtré mis sous pression.

Pour contrôler que les canaux de l'endoscope sont bien irrigués par de l'air sous pression, le système comporte avantageusement :
- un moyen pour mesurer la pression d'air injectée dans les canaux de l'endoscope,
- un moyen pour comparer la pression mesurée à une pression de calibrage initialement mesurée pour l'endoscope,
- un moyen pour générer une alerte lorsque la pression mesurée diffère de la pression de calibrage.

Un autre aspect de l'invention concerne un procédé pour stocker un endoscope médical, ledit procédé consistant à :
- placer l'endoscope dans un bac transportable,
- insérer le bac dans une étagère d'une armoire de stockage,
- raccorder les canaux de l'endoscope à un premier dispositif d'injection d'air de manière à ce que de l'air sous pression soit injecté dans lesdits canaux lorsque le bac est mis en place sur l'étagère,

Ce procédé est remarquable en ce qu'il consiste en outre à :
- fermer le bac, ce dernier étant pourvu d'un orifice d'entrée et d'un orifice de sortie,
- positionner l'orifice d'entrée en vis-à-vis d'un second dispositif d'injection d'air sous pression lorsque le bac est mis en place sur l'étagère,
- faire circuler un flux d'air dans le bac fermé, à l'extérieur de l'endoscope, depuis l'orifice d'entrée jusqu'à l'orifice de sortie, de manière à accélérer le séchage externe dudit endoscope.

Pour les mêmes raisons que celles décrites précédemment, ces étapes permettent de sécher et stocker les endoscopes dans des conditions extrêmement hygiéniques, avec des risques de contamination quasi nuls.

Pour réduire le volume d'air nécessaire au séchage externe de l'endoscope tout en limitant les risques de contamination, le procédé consiste en outre à :
- récupérer le flux d'air sous pression circulant dans le bac fermé,
- stériliser le flux d'air récupéré,
- réinjecter, dans le bac, le flux d'air stérilisé.

Selon une autre caractéristique avantageuse de l'invention permettant de contrôler une éventuelle perte d'étanchéité des endoscopes ou des bouchages de canaux, le procédé consiste en outre à :
- mesurer la pression d'air injectée dans les canaux de l'endoscope,
- comparer la pression mesurée à une pression de calibrage initialement mesurée pour l'endoscope,
- générer une alerte lorsque la pression mesurée diffère de la pression de calibrage.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 est une vue schématique montrant le système objet de l'invention dans son ensemble,
- la figure 2 est une vue schématique en coupe verticale du système objet de l'invention,
- la figure 3 est une vue en coupe verticale d'un bac de transport conforme à l'invention et installé sur une étagère de l'armoire de stockage,
- la figure 4 est une vue en coupe verticale d'un bac de transport dans une variante de réalisation et installé sur une étagère de l'armoire de stockage,
- la figure 5 est une vue en coupe verticale d'un bloc de connexion équipant les bacs de transport,
- la figure 6 est une vue de haut du bloc de connexion de la figure 5.

### Modes de réalisation de l'invention.

Le système objet de l'invention permet de sécher et stocker un ou plusieurs endoscopes médicaux après qu'ils aient été désinfectés à l'aide d'une solution désinfectante.

A la sortie de l'appareil de désinfection (LDE ou paillasse), l'endoscope est placé dans un bac transportable. En pratique, on utilise des bacs dédiés à chaque endoscope d'un service d'endoscopie de façon à réduire les infections croisées et protéger les endoscopes durant le transport. Un moyen de détrompage mécanique et/ou d'identification numérique (puce ou code-barres) ou visuelle (code couleur) permet d'empêcher ou d'avertir le personnel que le bac utilisé ne correspond pas à l'endoscope dédié. On pourra par exemple équiper chaque endoscope d'une puce ou d'un code-barres, les bacs 2 pouvant intégrer un moyen de lecture de ladite puce ou dudit code barre. Lorsque la puce ou le code-barres lus sur l'endoscope ne correspondent pas à la référence du bac, un signal d'alarme est automatiquement émis. On pourrait également prévoir d'attribuer une couleur unique à un couple endoscope/bac, pour éviter tout risque d'inversion.

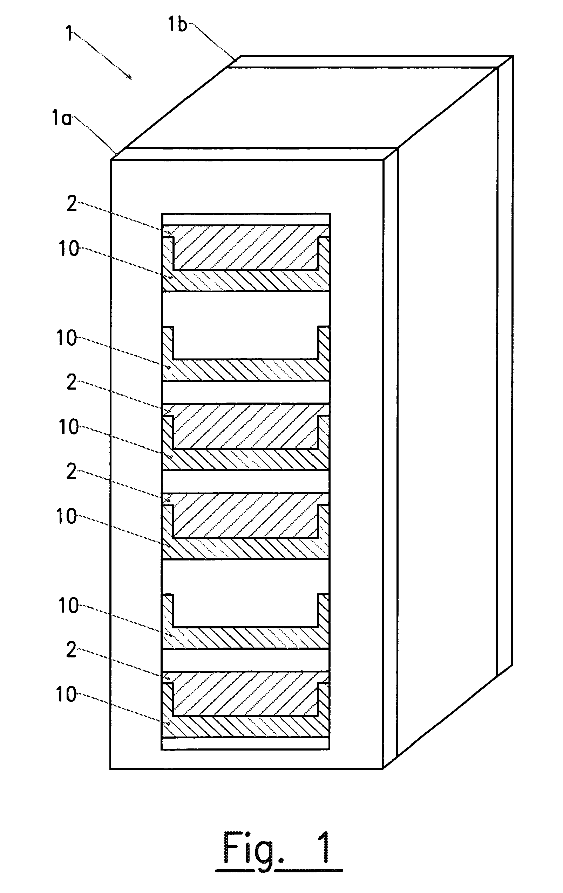

En se rapportant à la figure 1, le bac 2 est ensuite inséré dans une armoire destockage 1 pourvue d'au moins une étagère 10. En pratique, l"armoire 1 est pourvue de plusieurs étagères qui sont par exemple au nombre de six. L'armoire 1 est préférentiellement pourvue d'une porte 1a utilisée pour rentrer les bacs 2, et une autre porte 1 b pour les sortir.

Toujours dans le but de limiter les risques d'infections croisées, chaque étagère 10 est préférentiellement dédiée à un bac 2. Les étagères 10 peuvent par exemple être pourvues d'un moyen de détrompage mécanique et/ou d'un moyen d'identification permettant d'empêcher ou d'avertir le personnel qu'un bac 2 n'est pas positionné sur l'étagère 10 qui lui est dédiée. On pourra équiper chaque bac 2 d'une puce ou d'un code-barres, les étagères intégrant un moyen de lecture de ladite puce ou dudit code barre. Lorsque la puce ou le code-barres lus sur le bac ne correspondent pas à la référence de l'étagère, un signal d'alarme est automatiquement émis. On pourrait également prévoir d'attribuer une couleur unique à un couple bac/étagère, pour éviter tout risque d'inversion.

Les bacs 2 transportables utilisés sont réalisés en matière plastique ou en acier inoxydable. Ils sont sensiblement pleins, contrairement aux paniers décrits dans le document EP 0.986.988, et fermés par un couvercle 20. On utilise le terme « sensiblement pleins » car, comme cela est décrit plus après, certaines parois des bacs 2 peuvent comporter des ouvertures spécifiques de quelques mm² ou cm². L'endoscope se retrouve donc protégé par une enceinte close et isolé de l'environnement extérieur.

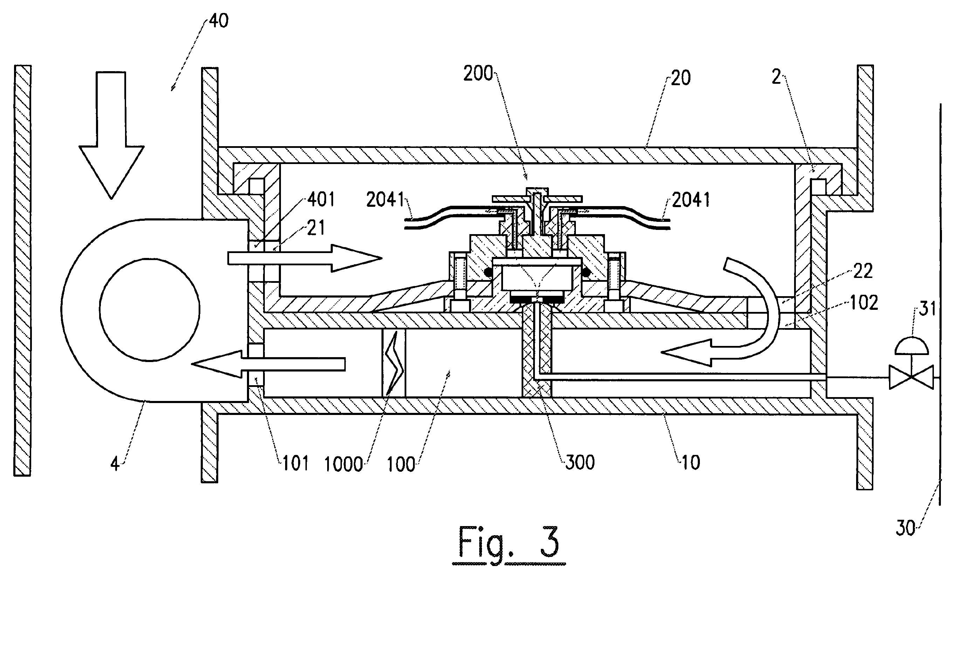

En se rapportant à la figure 3, les canaux de l'endoscope sont reliés à un bloc de connexion 200 agencé en position fixe dans le bac 2. Ce bloc de connexion 200 est avantageusement disposé sur la paroi de fond du bac 2. Il permet le raccordement des tubulures de désinfection et est compatible avec la majorité des tubulures de LDE et de paillasses. Le bloc 200 permet de retirer l'endoscope d'un LDE ou d'une paillasse, avec les tubulures utilisées pour la désinfection des canaux toujours en place sur ledit endoscope et de n'avoir à raccorder que l'extrémité standard sur ledit bloc.

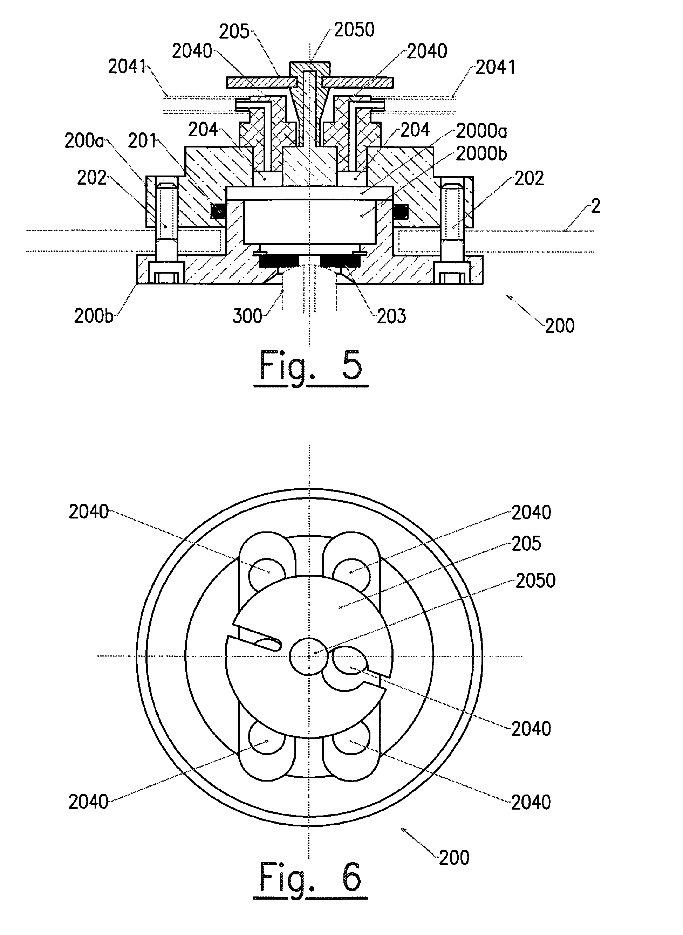

En se rapportant plus spécifiquement aux figures 5 et 6, le bloc de connexion 200 est formé de deux parties 200a et 200b. Ces deux parties sont disposées de part et d'autre de la paroi de fond du bac 2, la partie supérieure 200a du bloc étant disposée à l'intérieur dudit bac et la partie inférieure 200b à l'extérieur. Les deux parties 200a et 200b sont conformées pour s'emboiter l'une dans l'autre en enserrant la paroi du bac 2. Un joint torique 201 peut être prévu de manière à assurer un emboitement étanche. Le maintien en position des deux parties 200a-200b est par exemple réalisé au moyen de vis 202.

La partie inférieure 200b est pourvue d'un alésage 2000b débouchant dans un alésage 2000a réalisé dans la partie supérieure 200a. L'orifice d'entrée de l'alésage 2000b est obturé par un joint 203 percé en son centre et dont le fonctionnement sera décrit plus après dans la description. Le joint 203 est par exemple réalisé en caoutchouc souple.

La partie supérieure 200a du bloc de connexion est pourvue d'orifices 204 configuré pour recevoir l'extrémité standard 2040 les tubulures 2041 utilisées pour la désinfection des canaux. Les orifices 204 communiquent avec l'alésage 2000a. Un verrou 205 permet de bloquer les extrémités de connexion 2040 pour éviter qu'elles ne sortent des orifices 204 lors de l'injection d'air sous pression. Le verrou 205 se présente sous la forme d'un disque s'insérant et se bloquant sur une tige 2050 en saillie sur la partie supérieure 200a du bloc de connexion.

En pratique, il suffit de retirer l'endoscope d'un LDE ou d'une paillasse, avec les tubulures 2041 utilisées pour la désinfection des canaux toujours en place et d'insérer l'extrémité standard 2040 dans les orifices 204. Dès le retrait du LDE ou de la paillasse, l'endoscope se retrouve rapidement reconnecté et protégé par une enceinte close une fois le couvercle 20 du bac 2 refermé. L'endoscope peut alors être manipulé et transporté vers l'armoire de stockage 1, ou un autre lieu de stockage, en toute sécurité.

Une fois que l'endoscope est placé dans le bac 2, ce dernier est inséré dans une étagère 10 de l'armoire de stockage 1. Un premier dispositif d'injection d'air est raccordé aux canaux de l'endoscope de manière à ce que de l'air sous pression soit injecté dans lesdits canaux lorsque le bac 2 est mis en place sur l'étagère 10. De façon automatique, dès que le bac 2 est positionné sur l'étagère 10, le bloc de connexion 200 se raccorde de façon étanche à un circuit d'air comprimé (ci-après le premier dispositif d'injection d'air), sans que le personnel n'ait la moindre manipulation à faire sur l'endoscope, sur le couvercle 20 ou sur le bac 2. Dès que la porte d'entrée 1 a est refermée, les canaux de l'endoscope sont automatiquement ventilés avec de l'air traité. Chacun des canaux de chaque endoscope stockés dans chaque bac 2 sont ainsi séchés.

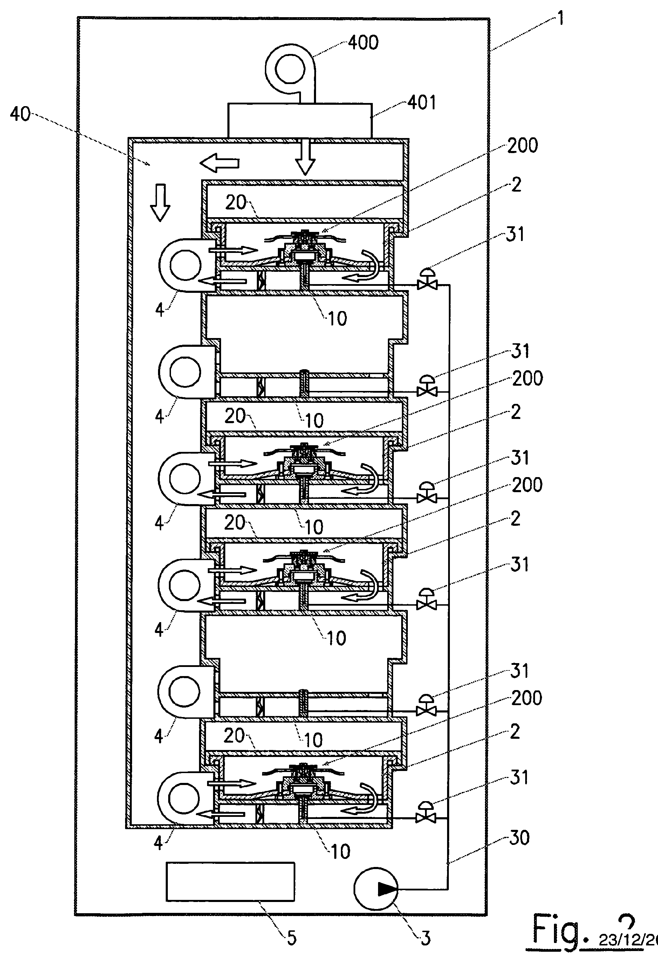

En se rapportant à la figure 2, le premier dispositif d'injection consiste en un compresseur 3 apte à délivrer de l'air sous pression à chaque étage de l'armoire 1 via un conduit 30. Un filtre type HEPA pourra être utilisé de manière à n'injecter que de l'air purifié. Des organes de contrôle 31, type vannes, pilotés automatiquement par un automate programmable et/ou une unité centrale, permettent de contrôler l'injection d'air au niveau de chaque étagère 10. A chaque étage, le conduit 30 débouche dans une tête d'injection 300 se connectant automatiquement au bloc de connexion 200 lorsque le bac 2 est mis en place sur une étagère 10.

En pratique, si le bloc de connexion 200 est disposé sur le fond du bac 2, alors la tête d'injection 300 est disposée en saillie sur la face supérieure de l'étagère 10 sur laquelle repose ledit bac. En se rapportant à la figure 3, lorsque le bac 2 est mis en place sur l'étagère 10, la tête d'injection 300 se positionne automatiquement en vis-à-vis de l'orifice d'entrée de l'alésage 2000b et se plaque de manière étanche contre le joint percé 203. La tête d'injection 300 est préférentiellement réalisée dans un matériau souple, type caoutchouc, de manière à assurer l'étanchéité au niveau du joint 203.

On pourrait prévoir une solution alternative (non représentée) dans laquelle le bloc de connexion 200 est disposé sur une des faces latérales du bac 2 ou au niveau du couvercle 200, la tête d'injection 300 étant positionnée dans l'armoire 1 de manière à se plaquer contre ledit bloc lorsque le bac est mis en place sur une des étagères 10.

Une fois que le bac 2 est positionné sur l'étagère 10, le compresseur 3 s'active automatiquement et la vanne 31 concernée s'ouvre, de manière à ce que de l'air sous pression (représenté en pointillés sur la figure 3) soit injecté dans les tubulures 2041 reliées aux canaux de l'endoscope, via le bloc de connexion 200 et la tête d'injection 300.

Chaque étage de l'armoire 1 est préférentiellement alimenté de manière cyclique par le premier dispositif d'injection d'air. Par exemple, en début de cycle, le bac 2 disposé sur l'étagère 10 la plus haute de l'armoire 1 sera alimenté en premier pendant un temps prédéterminé. Une fois que ce bac a cessé d'être alimenté, le bac disposé sur l'étagère du dessous sera à son tour alimenté pendant un temps prédéterminé. Et ainsi de suite jusqu'à ce que tous les bacs aient été alimentés par le premier dispositif d'injection. Le compresseur 3 peut être commandé de manière à ce que l'air soit injecté à des débits différents selon l'étage concerné.

Cette configuration permet de diagnostiquer d'éventuelles fuites des endoscopes ou que les canaux ne sont pas raccordés au bloc de connexion 200 ou que lesdits canaux sont bouchés. Par exemple, on paramètre initialement que pour un endoscope donné (associé à un bac 2 et à une étagère 10), on doit avoir 800 mbar de perte de charge. Si on détecte uniquement 750 mbar de perte de charge, c'est qu'il y a une fuite dans l'endoscope ou que les canaux ne sont pas (ou mal) raccordés au bloc de connexion 200. Au contraire, si on détecte 1000 mbar, c'est qu'un ou plusieurs canaux sont bouchés. Pour cette raison, la perte de charge de chaque endoscope est initialement mesurée. Cette perte de charge de référence correspond à une pression de calibrage (pour un endoscope donné, un débit d'injection X correspond à une pression Y).

Chaque étage de l'armoire 1 est pourvu d'un capteur de pression ou de tout autre moyen permettant de mesurer la pression d'air injectée dans les canaux de l'endoscope. Le capteur de pression est relié à une unité centrale équipée d'un processeur permettant de comparer la pression mesurée à la pression de calibrage. Lorsque la pression mesurée diffère de la pression de calibrage, l'unité centrale commande une alarme visuelle et/ou sonore indiquant que l'endoscope fuit ou que les canaux ne sont pas correctement raccordés au bloc de connexion 200 ou qu'il a un ou plusieurs canaux bouchés.

En se rapportant à la figure 3, le bac 2 comporte :
- un orifice d'entrée 21 se positionnant en vis-à-vis d'un second dispositif 4 d'injection d'air sous pression lorsque ledit bac est mis en place sur une des étagères 10 de l'armoire 1,
- un orifice de sortie 22 par lequel s'évacue l'air injecté.

Un flux d'air (schématisé par les flèches blanches sur les figures 2 et 3) peut alors circuler dans le bac 2, à l'extérieur de l'endoscope, depuis l'orifice d'entrée 21 jusqu'au dit orifice de sortie 22, de manière à accélérer le séchage externe dudit endoscope. Les orifices d'entrée 21 et de sortie 22 seront éloignés l'un de l'autre de manière à ce que le flux d'air sous pression puisse atteindre toutes les parties de l'endoscope. Le fond du bac 2 est avantageusement conçu de façon à ne laisser qu'un minimum de points de contact avec l'endoscope afin de permettre un séchage optimum de ce dernier lors de la circulation du flux d'air dans ledit bac.

En se rapportant à la figure 2, chaque étage de l'armoire 1 est pourvu d'un second dispositif 4 d'injection d'air. Ces seconds dispositifs 4 d'injection consistent en des ventilateurs installés à chaque étage de l'armoire 1, et fixés sur un montant de cette dernière. A chaque étage, l'orifice d'entrée 21 d'un bac 2 se positionne en vis à vis d'un orifice d'entrée 401 réalisé sur un montant de l'armoire 1 et communiquant avec un second dispositif 4 d'injection. Un joint pourra être prévu au niveau des orifices d'entrée 401 et/ou 21 de manière à assurer une certaine étanchéité au niveau de cette liaison.

Chacun des seconds dispositifs 4 d'injection communique avec une colonne d'air 40 mise sous pression par une turbine 400 qui aspire l'air ambiant. Un filtre 401, type HEPA, est placé en aval de la turbine 400 de manière à ce que l'air circulant dans la colonne 40 soit filtré et décontaminé. La pression de la colonne 40 est transmise aux bacs 2 via les seconds dispositifs 4 d'injection. Cet état des choses permet d'assurer une surpression dans chacun des bacs et donc d'empêcher toute contamination par de l'air externe au bac.

Selon une caractéristique avantageuse de l'invention permettant de réduire le volume d'air nécessaire au séchage externe de l'endoscope, l'orifice de sortie 22 du bac débouche dans une cavité 100 dans lequel le flux d'air sous pression est récupéré par le second dispositif d'injection 4 et réinjecté au niveau de l'orifice d'entrée 21 dudit bac.

Dans le mode préféré de réalisation représenté sur la figure 3, la cavité 100 est directement intégrée dans l'étagère 10 sur lequel est positionné le bac 2. L'orifice de sortie 22 du bac 2 se positionne en vis à vis d'un orifice d'entrée 102 de la cavité 100 lorsque ledit bac est mis en place sur ladite étagère. Un joint pourra être prévu au niveau des orifices 102 et/ou 22 de manière à assurer une certaine étanchéité au niveau de cette liaison. En pratique, l'orifice de sortie 22 est réalisé sur le fond du bac 2 et l'orifice d'entrée 102 est réalisé sur la face supérieure de l'étagère 10. Le fond du bac 2 est avantageusement incliné vers l'orifice de sortie 22, de sorte que les gouttes d'eau ruissellent naturellement dans la cavité 100 et ne soient plus en contact avec l'endoscope. La cavité 100 débouche, par l'intermédiaire d'un orifice de sortie 101, au niveau du second dispositif 4 d'injection.

En résumé, le second dispositif 4 injecte de l'air sous pression à l'intérieur du bac 2, via les orifices d'entrée 401 et 21. Le flux d'air sous pression traverse le bac 2 en réalisant un séchage externe de l'endoscope, puis s'échappe dudit bac par l'orifice 22 pour pénétrer dans la cavité 100 via l'orifice 102. Le flux d'air sort alors de la cavité 100, via l'orifice de sortie 101, pour être repris par le second dispositif 4 d'injection et réinjecté dans le bac 2.

En se rapportant à la figure 3, la cavité 100 intègre un moyen 1000 pour stériliser le flux d'air avant sa réinjection dans le bac 2. En pratique, on utilise une lampe UV au travers de laquelle passe le flux d'air avant d'être repris par le second dispositif 4 d'injection. Cette lampe UV permet de détruire d'éventuels germes encore présents dans le flux d'air après que ce dernier soit passé sur l'endoscope pour le sécher. D'autres moyens de stérilisation, tels que des filtres HEPA, pourront être utilisés en remplacement ou en complément de la lampe UV.

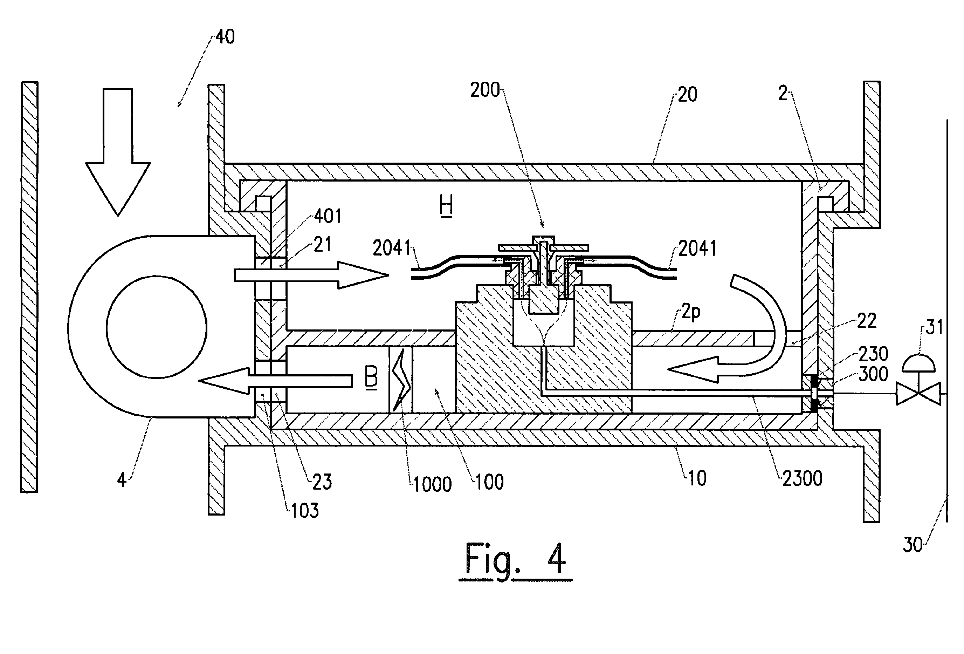

Dans une variante de réalisation représentée sur la figure 4, la cavité 100 est directement intégrée dans le bac 2. Dans ce cas, une plaque 2p sur laquelle est positionné l'endoscope divise le bac 2 en deux niveaux : un niveau haut H sur lequel est disposé l'endoscope et un niveau bas B formant la cavité 100. Cette plaque 2p est conçue de façon à ne laisser qu'un minimum de points de contact avec l'endoscope afin de permettre un séchage optimum de ce dernier lors de la circulation du flux d'air dans le bac 2. La plaque 2p comporte une ouverture 22 permettant au flux d'air de circuler du niveau haut H vers le niveau bas B. La plaque 2p peut être en outre pourvue de trous d'évacuation qui permettent aux gouttes d'eau de couler vers le niveau bas B et de ne plus être en contact avec l'endoscope.

Dans cette variante de réalisation, l'un des côtés latéraux du bac 2 dispose de deux orifices : un orifice 21, situé au niveau haut H, et permettant au second dispositif 4 d'injecter de l'air dans le bac 2, et un autre orifice 23, situé au niveau bas B, et permettant de récupérer le flux d'air circulant dans la cavité 100. De façon automatique, une fois le bac 2 mis en place dans l'armoire 1, chacun des deux orifices 21, 23 se positionne devant un couple d'orifices dédiés 401, 403 situés sur l'un des deux montants latéraux de l'armoire. Comme décrit précédemment, ces orifices 401, 403 coïncident avec les orifices d'injection et d'aspiration du second dispositif 4 d'injection. De l'air est ainsi pulsé dans le niveau haut H du bac 2, au travers de l'orifice supérieur 21. Le flux d'air pénètre dans le niveau bas B par l'ouverture 22 réalisée sur la plaque 2p et s'évacue par l'orifice inférieur 23. Ceci permet un mouvement d'air sur l'endoscope qui accélère son séchage externe.

Dans ce mode de réalisation, le bloc de connexion 200 est fixé sur la plaque 2p du bac 2 ou sur le fond dudit bac. Comme décrit précédemment, le bloc de connexion 200 est configuré pour recevoir l'extrémité standard des tubulures 2041 utilisées pour la désinfection des canaux. Un conduit 2300 permet de relier le bloc de connexion 200 au premier dispositif d'injection. L'extrémité de ce conduit 2300 est pourvue d'un joint percé 230 intégré dans une des parois latérales du bac 2. Une tête d'injection 300 est disposée en saillie sur un des montants de l'étagère 10 sur laquelle repose le bac 2. Lorsque ce dernier est mis en place sur l'étagère 10, la tête d'injection 300 se positionne automatiquement en vis-à-vis du joint 230 et se plaque de manière étanche contre ce dernier. La tête d'injection 300 est préférentiellement réalisée dans un matériau souple, type caoutchouc, de manière à assurer l'étanchéité au niveau du joint 203.

En se rapportant à la figure 2, la gestion de l'armoire 1 est assurée par un automate programmable 5 - intégrant éventuellement l'unité centrale mentionnée au paragraphe précédent - dans lequel sont enregistrés les temps de séchage de chacun des endoscopes, ce qui permet de pouvoir assurer une totale traçabilité pour chaque endoscope.

Une fois que le bac 2 est retiré de l'armoire 1 (par la porte de sortie 1b), un ticket est imprimé indiquant la date de début et de fin de stockage, ainsi que les données concernant l'endoscope. L'endoscope peut alors être transporté vers le lieu d'examen endoscopique. Afin de ne pas être souillé par l'endoscope une fois l'examen endoscopique terminé, le bac 2 peut être protégé par une enveloppe telle qu'une gaine plastique à usage unique et l'endoscope peut être à nouveau repositionné dans ledit bac pour son retour en salle de nettoyage. Le bac 2 protège alors le personnel contre d'éventuelles contaminations en provenance de l'endoscope.

## Revendications

1. Système pour sécher un endoscope médical, ledit système comportant :
- un bac (2) transportable dans lequel est placé l'endoscope,
- une armoire (1) de stockage pourvue d'au moins une étagère (10) sur laquelle est inséré le bac (2) dans lequel est placé l'endoscope,
- un premier dispositif (3, 30, 31) d'injection d'air raccordé aux canaux de l'endoscope de manière à ce que de l'air sous pression soit injecté dans lesdits canaux lorsque le bac (2) est mis en place sur l'étagère (10),
**se caractérisant par le fait que** le bac (2) est fermé et **par le fait que** ledit bac comporte :
- un orifice d'entrée (21) se positionnant en vis-à-vis d'un second dispositif (4) d'injection d'air sous pression lorsque ledit bac est mis en place sur l'étagère (10),
- un orifice de sortie (22) par lequel s'évacue l'air injecté, le flux d'air circulant dans le bac (2), à l'extérieur de l'endoscope, depuis l'orifice d'entrée (21) jusqu'au dit orifice de sortie (22), de manière à accélérer le séchage externe dudit endoscope.

2. Système selon la revendication 1, dans lequel l'orifice de sortie (22) du bac (2) débouche dans une cavité (100) dans lequel le flux d'air sous pression est récupéré par le second dispositif (4) d'injection et réinjecté au niveau de l'orifice d'entrée (21) dudit bac.

3. Système selon la revendication 2, dans lequel la cavité (100) est intégrée dans l'étagère (10) sur laquelle est positionné le bac (2), l'orifice de sortie (22) dudit bac se positionnant en vis à vis d'un orifice d'entrée (102) de ladite cavité lorsque ledit bac est mis en place sur ladite étagère.

4. Système selon la revendication 2, dans lequel la cavité (100) est directement intégrée dans le bac (2).

5. Système selon l'une des revendications 2 à 4, dans lequel la cavité (100) est pourvue d'un moyen (1000) pour stériliser le flux d'air avant sa reprise par le second dispositif (4) d'injection.

6. Système selon l'une des revendications précédentes, dans lequel les canaux de l'endoscope sont reliés à un bloc de connexion (200) agencé en position fixe dans le bac (2), le premier dispositif (3, 30, 31) d'injection d'air étant pourvu d'une tête d'injection (300) se connectant automatiquement audit bloc de connexion lorsque ledit bac est mis en place sur l'étagère (10).

7. Système selon la revendication 6, dans lequel le bloc de connexion (200) est disposé sur le fond du bac (2) et la tête d'injection (300) est disposée en saillie sur la face supérieure de l'étagère (10) sur laquelle repose ledit bac.

8. Système selon l'une des revendications précédentes, dans lequel l'armoire (1) de stockage comporte plusieurs étagères (10) destinées à recevoir chacune un bac (2), chaque bac étant dédié à un endoscope et chaque étagère étant dédiée à un bac.

9. Système selon la revendication 8, dans lequel les étagères (10) sont pourvues d'un moyen de détrompage mécanique et/ou d'un moyen d'identification permettant d'empêcher ou d'avertir qu'un bac (2) n'est pas positionné sur l'étagère qui lui est dédiée.

10. Système selon l'une des revendications 8 ou 9, dans lequel chaque étage de l'armoire (1) de stockage est alimenté de manière cyclique par le premier dispositif (3, 30, 31) d'injection d'air.

11. Système selon l'une des revendications 8 à 10, dans lequel chaque étage de l'armoire (1) de stockage est pourvu d'un second dispositif (4) d'injection d'air sous pression destiné à faire circuler un flux d'air sous pression dans les bacs fermés, lesdits seconds dispositifs d'injection d'air communiquant avec une même colonne (40) d'air filtré mis sous pression.

12. Système selon l'une des revendications précédentes, comportant :
- un moyen pour mesurer la pression d'air injectée dans les canaux de l'endoscope,
- un moyen pour comparer la pression mesurée à une pression de calibrage initialement mesurée pour l'endoscope,
- un moyen pour générer une alerte lorsque la pression mesurée diffère de la pression de calibrage.

13. Procédé pour stocker un endoscope médical, ledit procédé consistant à :
- placer l'endoscope dans un bac (2) transportable,
- insérer le bac (2) dans une étagère (10) d'une armoire(1) de stockage,
- raccorder les canaux de l'endoscope à un premier dispositif (3, 30, 31) d'injection d'air de manière à ce que de l'air sous pression soit injecté dans lesdits canaux lorsque le bac (2) est mis en place sur l'étagère (10),
**se caractérisant par le fait qu'**il consiste en outre à :
- fermer le bac (2), ce dernier étant pourvu d'un orifice d'entrée (21) et d'un orifice de sortie (22),
- positionner l'orifice d'entrée en vis-à-vis d'un second dispositif (4) d'injection d'air sous pression lorsque le bac (2) est mis en place sur l'étagère (10),
- faire circuler un flux d'air dans le bac (2) fermé, à l'extérieur de l'endoscope, depuis l'orifice d'entrée (21) jusqu'à l'orifice de sortie (22), de manière à accélérer le séchage externe dudit endoscope.

14. Procédé selon la revendication 13, consistant à :
- récupérer le flux d'air sous pression circulant dans le bac (2) fermé,
- stériliser le flux d'air récupéré,
- réinjecter, dans le bac (2), le flux d'air stérilisé.

15. Procédé selon l'une des revendications 13 ou 14, consistant à :
- mesurer la pression d'air injectée dans les canaux de l'endoscope,
- comparer la pression mesurée à une pression de calibrage initialement mesurée pour l'endoscope,
- générer une alerte lorsque la pression mesurée diffère de la pression de calibrage.

## Claims

1. System for drying a medical endoscope, said system comprising:
- a portable tank (2) in which the endoscope is placed,
- a storage cabinet (1) provided with at least one shelf (10) on which the tank (2) in which the endoscope is placed is inserted,
- a first air injection device (3, 30, 31) connected to the canals of the endoscope in such a way that pressurized air is injected into said canals when the tank (2) is in place on the shelf (10),
**characterized in that** the tank (2) is closed, and **in that** said tank comprises:
- an inlet orifice (21) positioned facing a second pressurized-air injection device (4) when said tank is in place on the shelf (10),
- an outlet orifice (22) through which the injected air is removed, the air stream flowing through the tank (2), on the outside of the endoscope, from the inlet orifice (21) as far as said outlet orifice (22), in such a way as to accelerate the external drying of said endoscope.

2. System according to Claim 1, in which the outlet orifice (22) of the tank (2) leads into a cavity (100) in which the stream of pressurized air is recovered by the second injection device (4) and is re-injected at the inlet orifice (21) of said tank.

3. System according to Claim 2, in which the cavity (100) is integrated in the shelf (10) on which the tank (2) is positioned, the outlet orifice (22) of said tank being positioned facing an inlet orifice (102) of said cavity when said tank is in place on said shelf.

4. System according to Claim 2, in which the cavity (100) is integrated directly in the tank (2).

5. System according to one of Claims 2 to 4, in which the cavity (100) is provided with a means (1000) for sterilizing the air stream before the latter is recovered by the second injection device (4).

6. System according to one of the preceding claims, in which the canals of the endoscope are attached to a connector block (200) arranged in a fixed position in the tank (2), the first air injection device (3, 30, 31) being provided with an injection head (300) which connects automatically to said connector block when said tank is in place on the shelf (10).

7. System according to Claim 6, in which the connector block (200) is arranged on the bottom of the tank (2), and the injection head (300) is arranged protruding from the upper face of the shelf (10) on which said tank rests.

8. System according to one of the preceding claims, in which the storage cabinet (1) comprises several shelves (10), each intended to receive one tank (2), each tank being dedicated to one endoscope, and each shelf being dedicated to one tank.

9. System according to Claim 8, in which the shelves (10) are provided with a mechanical foolproofing means and/or an identification means for ensuring that a tank (2) is not positioned on the shelf not dedicated to it.

10. System according to either of Claims 8 and 9, in which each level of the storage cabinet (1) is supplied cyclically via the first air injection device (3, 30, 31).

11. System according to one of Claims 8 to 10, in which each level of the storage cabinet (1) is provided with a second pressurized-air injection device (4) intended to circulate a stream of pressurized air through the closed tanks, said second air injection devices communicating with a same column (40) of filtered air under pressure.

12. System according to one of the preceding claims, comprising:
- a means for measuring the air pressure injected in the canals of the endoscope,
- a means for comparing the measured pressure to a reference pressure initially measured for the endoscope,
- a means for generating an alarm when the measured pressure differs from the reference pressure.

13. Method for storing a medical endoscope, said method consisting in:
- placing the endoscope in a portable tank (2),
- inserting the tank (2) into a shelf (10) of a storage cabinet (1),
- connecting the canals of the endoscope to a first air injection device (3, 30, 31) in such a way that pressurized air is injected into said canals when the tank (2) is in place on the shelf (10),
**characterized in that** said method additionally consists in:
- closing the tank (2), the latter being provided with an inlet orifice (21) and an outlet orifice (22),
- positioning the inlet orifice facing a second pressurized-air injection device (4) when the tank (2) is in place on the shelf (10),
- circulating an air stream through the closed tank (2), on the outside of the endoscope, from the inlet orifice (21) as far as the outlet orifice (22), in such a way as to accelerate the external drying of said endoscope.

14. Method according to Claim 13, consisting in:
- recovering the stream of pressurized air flowing in the closed tank (2),
- sterilizing the recovered air stream,
- re-injecting the sterilized air stream into the tank (2).

15. Method according to either of Claims 13 and 14, consisting in:
- measuring the air pressure injected in the canals of the endoscope,
- comparing the measured pressure to a reference pressure initially measured for the endoscope,
- generating an alarm when the measured pressure differs from the reference pressure.

## Patentansprüche

1. System zum Trocknen eines medizinischen Endoskops, wobei das System aufweist:
- einen transportierbaren Behälter (2), in dem das Endoskop angeordnet ist,
- einen Lagerschrank (1), der mit mindestens einem Regalbrett (10) versehen ist, auf das der Behälter (2) eingefügt wird, in dem das Endoskop angeordnet ist,
- eine erste Lufteinspritzvorrichtung (3, 30, 31), die mit den Kanälen des Endoskops verbunden wird, damit Druckluft in die Kanäle eingespritzt wird, wenn der Behälter (2) auf dem Regalbrett (10) angeordnet ist,
**dadurch gekennzeichnet, dass** der Behälter (2) geschlossen ist, und dadurch, dass der Behälter aufweist:
- eine Eingangsöffnung (21), die gegenüber einer zweiten Drucklufteinspritzvorrichtung (4) positioniert wird, wenn der Behälter auf dem Regalbrett (10) angeordnet wird,
- eine Ausgangsöffnung (22), durch die die eingespritzte Luft abgeführt wird, wobei der Luftstrom im Behälter (2) außerhalb des Endoskops von der Eingangsöffnung (21) bis zur Ausgangsöffnung (22) zirkuliert, um das äußere Trocknen des Endoskops zu beschleunigen.

2. System nach Anspruch 1, bei dem die Ausgangsöffnung (22) des Behälters (2) in einen Hohlraum (100) mündet, in dem der Druckluftstrom von der zweiten Einspritzvorrichtung (4) wiedergewonnen und erneut im Bereich der Eingangsöffnung (21) des Behälters eingespritzt wird.

3. System nach Anspruch 2, bei dem der Hohlraum (100) in das Regalbrett (10) integriert ist, auf dem der Behälter (2) positioniert wird, wobei die Ausgangsöffnung (22) des Behälters gegenüber einer Eingangsöffnung (102) des Hohlraums positioniert wird, wenn der Behälter auf dem Regalbrett angeordnet wird.

4. System nach Anspruch 2, bei dem der Hohlraum (100) direkt in den Behälter (2) integriert ist.

5. System nach einem der Ansprüche 2 bis 4, bei dem der Hohlraum (100) mit einer Einrichtung (1000) versehen ist, um den Luftstrom vor seiner Wiederaufnahme durch die zweite Einspritzvorrichtung (4) zu sterilisieren.

6. System nach einem der vorhergehenden Ansprüche, bei dem die Kanäle des Endoskops mit einem Anschlussblock (200) verbunden werden, der in ortsfester Stellung im Behälter (2) angeordnet ist, wobei die erste Lufteinspritzvorrichtung (3, 30, 31) mit einem Einspritzkopf (300) versehen ist, der automatisch an den Anschlussblock angeschlossen wird, wenn der Behälter auf dem Regalbrett (10) angeordnet wird.

7. System nach Anspruch 6, bei dem der Anschlussblock (200) hinten im Behälter (2) und der Einspritzkopf (300) an der Oberseite des Regalbretts (10) vorstehend angeordnet ist, auf dem der Behälter ruht.

8. System nach einem der vorhergehenden Ansprüche, bei dem der Lagerschrank (1) mehrere Regalbrette (10) aufweist, die dazu bestimmt sind, jedes einen Behälter (2) aufzunehmen, wobei jeder Behälter für ein Endoskop und jedes Regalbrett für einen Behälter bestimmt ist.

9. System nach Anspruch 8, bei dem die Regalbretter (10) mit einer mechanischen Unverwechselbarkeitseinrichtung und/oder einer Identifikationseinrichtung versehen sind, die es ermöglichen, zu verhindern oder darauf aufmerksam zu machen, dass ein Behälter (2) nicht auf dem für ihn bestimmten Regalbrett positioniert ist.

10. System nach einem der Ansprüche 8 oder 9, bei dem jedes Regalbrett des Lagerschranks (1) zyklisch von der ersten Lufteinspritzvorrichtung (3, 30, 31) gespeist wird.

11. System nach einem der Ansprüche 8 bis 10, bei dem jedes Regalbrett des Lagerschranks (1) mit einer zweiten Drucklufteinspritzvorrichtung (4) versehen ist, die dazu bestimmt ist, einen Druckluftstrom in den geschlossen Behältern zirkulieren zu lassen, wobei die zweiten Lufteinspritzvorrichtungen mit der gleichen Säule gefilterter Druckluft (40) in Verbindung stehen.

12. System nach einem der vorhergehenden Ansprüche, das aufweist:
- eine Einrichtung zum Messen des in die Kanäle des Endoskops eingespritzten Luftdrucks,
- eine Einrichtung zum Vergleich des gemessenen Drucks mit einem Kalibrierdruck, der anfangs für das Endoskop gemessen wird,
- eine Einrichtung zur Erzeugung eines Alarms, wenn der gemessene Druck sich von dem Kalibrierdruck unterscheidet.

13. Verfahren zum Lagern eines medizinischen Endoskops, wobei das Verfahren darin besteht:
- das Endoskop in einem transportablen Behälter (2) anzuordnen,
- den Behälter (2) auf ein Regalbrett (10) eines Lagerschranks (1) einzufügen,
- die Kanäle des Endoskops mit einer ersten Lufteinspritzvorrichtung (3, 30, 31) zu verbinden, damit Druckluft in die Kanäle eingespritzt wird, wenn der Behälter (2) auf dem Regalbrett (10) angeordnet wird,
**dadurch gekennzeichnet, dass** es außerdem darin besteht:
- den Behälter (2) zu schließen, wobei dieser mit einer Eingangsöffnung (21) und einer Ausgangsöffnung (22) versehen ist,
- die Eingangsöffnung gegenüber einer zweiten Drucklufteinspritzvorrichtung (4) anzuordnen, wenn der Behälter (2) auf dem Regalbrett (10) angeordnet wird,
- einen Luftstrom im geschlossenen Behälter (2) außerhalb des Endoskops von der Eingangsöffnung (21) bis zur Ausgangsöffnung (22) zirkulieren zu lassen, um das äußere Trocknen des Endoskops zu beschleunigen.

14. Verfahren nach Anspruch 13, das darin besteht:
- den im geschlossenen Behälter (2) zirkulierenden Druckluftstrom wiederzugewinnen,
- den wiedergewonnenen Luftstrom zu sterilisieren,
- den sterilisierten Luftstrom wieder in den Behälter (2) einzuspritzen.

15. Verfahren nach einem der Ansprüche 13 oder 14, das darin besteht:
- den in die Kanäle des Endoskops eingespritzten Luftdruck zu messen,
- den gemessenen Druck mit einem anfangs für das Endoskop gemessenen Kalibrierdruck zu vergleichen,
- einen Alarm zu erzeugen, wenn der gemessene Druck sich vom Kalibrierdruck unterscheidet.
